# EUROPEAN PATENT APPLICATION

(11) **EP 4 026 615 A1**
(43) Date of publication of application: **13.07.2022**
(21) Application number: 21150910.4
(22) Date of filing: 11.01.2021
(51) Int. Cl.: B01L 3/00

(54) **MICROFLUIDIC CIRCUIT, MICROFLUIDIC CHIP, KIT AND METHOD FOR ISOLATING AND PURIFYING AN ANALYTE FROM A BIOLOGIC SAMPLE**

(71) Applicant: Curiosity Diagnostics sp. z o.o, 01-796 Warsaw (PL)
(72) Inventor: Gewartowski, Kamil Robert, 02-127 Warsaw (PL); Garstecki, Piotr, 01-494 Warsaw (PL); Lirski, Maciej, 05-077 Warsaw (PL); Wisniewski, Krzysztof, 05-800 Pruszków (PL); Debski, Pawel, 03-984 Warsaw (PL)
(74) Representative: Prinz & Partner mbB

(57) **Abstract**

The present invention relates to a microfluidic circuit, microfluidic chip, kit and method for isolating and purifying an analyte, preferably a nucleic acid, according to the independent claims.

## Description

### TECHNICAL FIELD:

The present invention relates to a microfluidic circuit, microfluidic chip, kit and method for isolating and purifying an analyte, preferably a nucleic acid, according to the independent claims.

### BACKGROUND ART:

The field of analyzing nucleic acids using enzymatic reactions, such as in a polymerase chain reaction assay, generally requires the use of reaction enzymes and/or proteins which are very sensitive to reagents commonly used during the preceding nucleic acid isolation step, in particular isolation of DeoxyriboNucleic Acid (DNA) and RiboNucleic Acid (RNA).

The reagents commonly used in the isolation step facilitate lysis of the biological samples including biologic cells and/or virus material. In other words, the reagents facilitate that the outer boundary or cell membrane / virus wall is at least in part broken down or destroyed in order to release inter-cellular / inter-viral material, such as DNA, RNA, protein or organelles from a cell. Such a destructure requires suitable strong lysis agents, such as guanidine hydrochloride, alcohol, e.g., ethanol, isopropanol, or sodium dodecyl sulfate (SDS). The lysis agent may be used in form of a lysis buffer mixture, such as the SDS lysis buffer, which may comprise 0.5% (w/v) SDS, 0.05 M Tris·Cl, Adjust pH to 8.0 and add 1 mM fresh dithiothreitol (DTT).

Due to the strong lysis effect on peptidic structures, these lysis agents may, however, interfere with enzymes to be used for the analyzing step of nucleic acids, such as required for the polymerase chain reaction (PCR). Thus, there is a need for providing devices, which reduce the remaining lysis agent contamination prior to conducting the analyzing step.

In addition to lysis agents, washing agents used to wash and purify the isolated nucleic acid from the remaining cellular/wall fragments may also interfere with the enzymatic reaction on nucleic acids, such as PCR, in particular in case the washing agent is selected from a suitable alcohol, such as ethanol or isopropanol.

Generally, the lysate comprising the destroyed cells / virus, isolated nucleic acids and lysis agent is contacted with capturing agents, such as beads, preferably micro beads, in order to bind the nucleic acid to the beads and to, thus, isolate the bound nucleic acid beads from the destroyed cells / virus and further released inter-cellular/ inter-viral material.

In case the enzymatic reaction to analyze the nucleic acid is performed on a microfluidic device, such as a microfluidic chip, the lysis agent and the washing agent have to be separated from the isolated nucleic acid, which is usually conducted by transferring it to a waste chamber, which also can be any outlet of the microfluidic device, whereas the isolated and eluted nucleic acid has to be transferred to a reaction chamber, where the enzymatic analyzing reaction takes place.

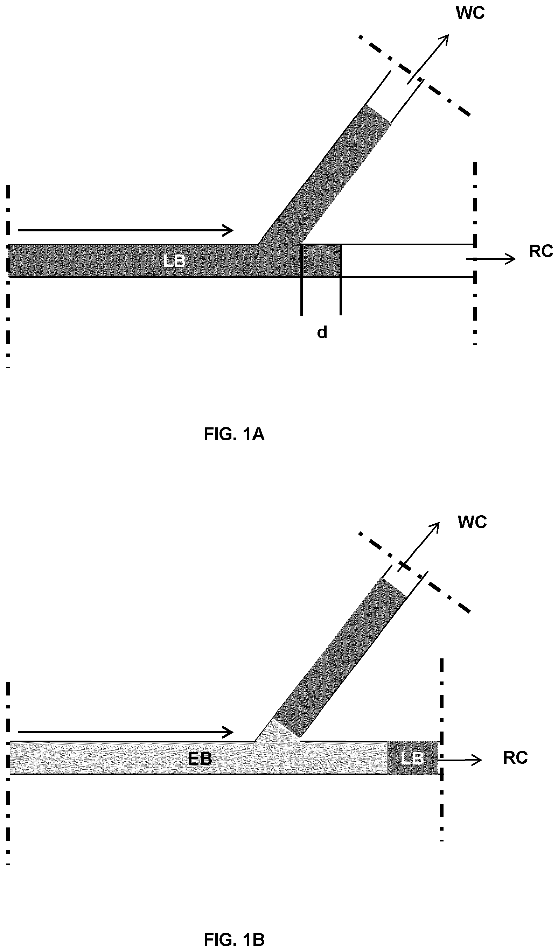

To facilitate the separation, the microfluidic chip generally comprises a channel system including at least one junction at which the channel branches into a sub-channel connecting in fluidic flow down-stream direction to the waste chamber and a sub-channel connecting in fluidic flow down-stream direction to the reaction chamber. The channel system comprises suitable means, such as two-state valves, to facilitate fluidic flow in direction to the waste chamber (WC) or to facilitate fluidic flow in direction to the reaction chamber (RC). As these valves cannot be arranged directly on the junction, at least some lysate buffer (LB), which is to be transferred in flow direction downstream (fd) to the waste chamber (WC), also enters at least part (distance d) of the sub-channel connecting to the reaction chamber (RC), which is illustrated in figure 1A (valves are not illustrated). When in a next step nucleic acid is eluted with an elution buffer (EB) in flow direction downstream (fd) to the reaction chamber (RC), then the remaining part of lysate buffer (LB) and/or remaining washing agent will be also transferred to the reaction chamber and may interfere with the enzymatic reaction on the nucleic acids (see figure 1B) and thereby reducing the analytical precision.

Thus, there exists an ongoing need to increase analytical precision of enzymatic reactions on analytes of biologic samples, preferably nucleic acids, such as PCR.

Furthermore, there exists an ongoing need in providing simple and cost effective isolation and purification processes of analytes of biologic samples, preferably nucleic acids, which reduce or prevent remaining contamination in particular of lysis agents in the following enzymatic reactions.

Thus, it is an aim of the present invention to provide an alternative and/or improved microfluidic device, such as microfluidic circuit or microfluidic chip for isolating and purifying analytes, preferably nucleic acids from biologic samples providing a suitable analytical precision and/or reducing or preventing contamination with contamination agents, such as lysate agents and/or washing agents in the enzymatic analyzing reactions on the analytes, preferably nucleic acids, more preferably wherein the isolation and purification processes are simple and cost effective.

### SUMMARY OF THE INVENTION:

One or more problems of the present invention is/are solved by the subject matter of the independent claims, namely a microfluidic circuit / a microfluidic chip / a kit / a system for isolating and purifying analytes, preferably nucleic acids from biologic samples, and uses thereof, as well as a process for controlling fluidic flow in an isolation and purification reaction, and a process for isolating and purifying an analyte, preferably a nucleic acid, from a biological cell sample. Advantages (preferred embodiments) are set out in the detailed description hereinafter and/or the accompanying figures as well as in the dependent claims.

Accordingly, a first aspect of the present invention relates to a microfluidic circuit for use in a microfluidic chip for isolating and purifying an analyte, preferably a nucleic acid, comprised in a biologic sample, wherein the microfluidic circuit is arranged in a substrate and comprises a first microfluidic channel (MC1) fluidicly connecting an outlet of a buffer chamber (BC) with an inlet of an elution chamber (EC) and a second microfluidic channel (MC2) fluidicly connecting an outlet of the elution chamber (EC) with an inlet of a waste chamber (WC) and with a or connectable to a reaction chamber (RC), wherein the second microfluidic channel (MC2) is branched at a first junction (J1) into two downstream sub-channels (MC21, MC22) of a first generation, wherein the first sub-channel (MC21) connects to the inlet of the waste chamber (WC) and the second sub-channel (MC22) connects or is connectable to the reaction chamber (RC), characterized in that the second sub-channel (MC22) of first generation is branched at a second junction (J2) into two downstream sub-channels of second generation, wherein a first sub-channel (MC221) connects or is connectable to the reaction chamber (RC) and a second sub-channel (MC222) is connected to the buffer chamber (BC) or to a further buffer chamber (BC) and is configured to facilitate fluidic flow in at least part of the second sub-channel of first generation (MC22) and the second microfluidic channel (MC2) in direction to the outlet of the elution chamber (EC).

According to a second aspect of the present invention, a microfluidic chip for isolating and purifying an analyte, preferably a nucleic acid, comprised in a biologic sample is provided having a sample chamber (SC) fluidicly connected with an inlet of a capturing chamber (CC), characterized in that an outlet of the capturing chamber (CC) is fluidicly connected to the microfluidic circuit according to first aspect of the present invention, wherein the outlet of the capturing chamber (CC) is connected with a third microfluidic channel (MC3) to a fourth junction (J4) of the first microfluidic channel (MC1).

According to a third aspect of the present invention, a kit is provided comprising or consisting of the microfluidic chip according to the second aspect of the present invention, and one or more, the same or different, suitable reaction agents selected from the group consisting of a reagent suitable for performing lysis of biologic sample (lysis agent); a reagent suitable for performing washing of lysed biologic cell fragments from the isolated analyte, preferably nucleic acid, respectively bound to at least part of the capturing carriers (washing agent); a reagent suitable for eluting the isolated analyte, preferably nucleic acid, from the capturing carriers (elution agent); a reagent suitable for suspending the sample (dissolution agent).

According to a fourth aspect of the present invention, a system configured to perform isolation and purification of an analyte, preferably a nucleic acid, from a biologic sample is provided, characterized in that it comprises or consists of a microfluidic chip according to the second aspect of the present invention and a thermocycler.

According to a fifth aspect of the present invention, a process for isolating and purifying an analyte, preferably a nucleic acid, from a biological cell sample comprising or consisting of the following steps is provided:
a. Providing the system of the fourth inventive aspect, providing a biological sample, providing a lysis agent, providing an elution agent and optionally providing a washing agent,
b. Loading the biological sample into the sample chamber (SC) of the microfluidic chip,
c. Subsequently mixing the lysis agent with the biological sample thereby providing a fluidic mixture comprising lysed biologic sample fragments and isolated analyte, preferably nucleic acid,
d. Subsequently passing the fluidic mixture of step c) to the capturing chamber (CC) comprising the magnetic capturing carriers so that the isolated analyte, preferably nucleic acid, binds to at least part of the magnetic capturing carriers,
e. Subsequently passing the fluidic mixture of step d) via the third microfluidic channel (MC3) to the elution chamber (EC), wherein the magnetic field excitation means excites a suitable magnetic field so that at least part of the magnetic capturing carriers agglomerate as a magnetic conglomerate, which is preferably located due to the applied magnetic field at a wall of the elution chamber (EC),
f. Subsequently passing the elution agent from the buffer chamber (BC) via the elution chamber (EC) to the waste chamber (WC), wherein the magnetic field is maintained to keep the magnetic conglomerate located at the wall of the eluting chamber (EC),
g. Subsequently passing the elution agent from the buffer chamber (BC) to the second sub-channel (MC222) to facilitate fluidic flow in at least part of the second sub-channel of first generation (MC22) and the second microfluidic channel (MC2) towards the outlet of the elution chamber (EC) in order to remove remaining lysis agent from the second sub-channel of first generation (MC22), wherein the magnetic field is maintained to keep the magnetic conglomerate located at the wall of the eluting chamber (EC),
h. Subsequently passing the elution agent from the buffer chamber (BC) via the elution chamber (EC) to the waste chamber (WC) in order to remove the remaining lysis agent from the second micro fluidic channel (MC2), wherein the magnetic field is maintained to keep the magnetic conglomerate located at the wall of the eluting chamber (EC),
i. Subsequently removing the magnetic field and exciting the aggregated conglomerate of magnetic capture carriers with a suitable ultrasound impulse in order to separate the magnetic capturing carriers and thereby eluting the isolation analyte, preferably nucleic acid, from the capturing carriers, and
j. Subsequently passing the elution agent from the buffer chamber via the elution chamber (EC) to the reaction chamber (RC) thereby moving the eluted analyte, preferably nucleic acid, to the reaction chamber (RC).

According to a fifth aspect of the present invention, a use of a microfluidic circuit according to the first aspect of the present invention or a microfluidic chip according to the second aspect of the present invention or a kit according to the third aspect of the present invention or a system according to the fourth aspect of the present invention for isolating and purifying an analyte, preferably a nucleic acid, from a biological sample is provided, preferably for conducting a biochemical assay selected from the group consisting of an enzymatic analysis of analytes of biological samples, preferably nucleic acids including DNA or RNA analysis, such as a polymerase chain reaction (PCR) and in particular a PCR with high-throughput sequencing.

The inventive aspects of the present invention as disclosed hereinbefore can comprise any possible (sub-)combination of the preferred inventive embodiments as set out in the dependent claims or as disclosed in the following detailed description and/or in the accompanying figures, provided the resulting combination of features is reasonable to a person skilled in the art.

### BRIEF DESCRIPTION OF THE DRAWINGS:

Further characteristics and advantages of the present invention will ensue from the accompanying drawings, wherein
Figs. 1A, 1B represent schematic top views of part of a prior art microfluidic channel system at a junction connecting to the waste chamber (WC) and connecting to the reaction chamber (RC)
Figs. 2A, 2B represent schematic top views of an inventive microfluidic circuit 1 comprising a buffer chamber 14 (BC), an elution chamber 15 (EC), a waste chamber 16 (WC) and a reaction chamber 17 (RC)
Figs. 3A - 3F represent schematic top views of an exploded part of an inventive microfluidic circuit 1 in the area of the first and second junction (J1, J2) of the second microfluidic channel 12 respectively illustrating the fluidic flow condition of the elution buffer (EB) and the lysate agent buffer (LB)
Fig. 4 represents a schematic top view of an inventive microfluidic circuit 1 comprising a buffer chamber 14 (BC), an elution chamber 15 (EC), a waste chamber 16 (WC) and a reaction chamber 17 (RC) and a channel bypassing the elution chamber 15 (EC)
Fig. 5 represents a schematic top view of an inventive microfluidic chip 3 comprising a sample chamber 19 (SC), a capturing chamber 18 (CC) connected to the inventive microfluidic circuit 1 of Fig. 3 and
Fig. 6 represents a schematic top view of a design of an inventive microfluidic chip 2.

### DETAILED DESCRIPTION OF THE INVENTION:

As set out in more detail hereinafter, the inventors of the different aspects of the present invention have found out that the inventive microfluidic circuit facilitates removing all residual contaminants, such as lysate agent and washing buffer, that could otherwise inhibit further reaction of the isolated analyte, preferably nucleic acid, in particular in an enzymatic analyzing reaction, such as polymerase chain reaction (PCR).

Furthermore, the use of magnetic capturing carriers for binding to the nucleic acid, isolated from the lysed biologic sample is advantageous, as the isolated analyte, preferably nucleic acid, respectively bound the magnetic capturing carriers, such as magnetic (micro) beads, can be located upon magnetic field excitation in the area of the eluting chamber (EC), preferably at least at part of the wall of the eluting chamber (EC).

Generally a magnetic field is used to agglomerate the analyte, preferably nucleic acid, bound to the magnetic capturing carriers in such a way that they form a magnetic conglomerate. This means, that the analyte, preferably nucleic acid, is predominantly integrated into the magnetic conglomerate so that any contamination agents, such as lysis and/or washing agents or elution agents are substantially hindered to integrate within the agglomerated analyte, preferably nucleic acid, bound conglomerate. In other words, the excitation of the magnetic field, which is generally unwanted due to its agglomeration effects, surprisingly reduces, preferably prevents unwanted analyte, preferably nucleic acid, elution from the magnetic capturing carriers during washing procedures. Thus, the precision of the analytical reaction, preferably analytical nucleic acid reaction, is increased by use of the magnetic capturing carriers. According to the present invention, the inventors could show that only about 5 wt.% of the analyte, in particular nucleic acid, respectively bound to the magnetic capturing carriers are washed to the waste chamber. This reduced amount of analyte, preferably nucleic acid, loss is in particular advantageous as all residual lysing and washing agents, which may act as inhibitors to the further enzymatic procedure on the analyte, preferably nucleic acids, are at the same time also removed to the waste chamber.

In order to elute the analyte, preferably nucleic acid, respectively bound to the magnetic capture carriers, the present invention teaches to excite the agglomerated analyte, preferably nucleic acid, bound capture carrier conglomerate with a suitable ultrasound impulse. This suitable ultrasound impulse destroys the agglomerated analyte, preferably nucleic acid, bound magnetic conglomerate and results in separated magnetic capture carriers respectively bound with analyte, preferably nucleic acid, suspended in the suitable elution agent, wherein the elution agent can efficiently elute the analyte, preferably nucleic acid, from the magnetic capture carriers.

In accordance with the present invention the term "analyte" includes suitable analytes to be isolated from biologic sample, preferably peptidic analytes or nucleic acid analytes or bacterial or viral analytes. Preferably, the analyte according to the present invention is regarded a nucleic acid analyte to be isolated from a biologic sample. Furthermore, in case the present invention uses the term "nucleic acid" also refers to any other kind of suitable analyte to be isolated and purified from a biologic sample unless otherwise indicated.

The expression "microfluidic circuit" means according to the present invention that a microfluidic channel system with suitable chambers is arranged in a suitable substrate. At least the microfluidic channels and the elution chamber of the inventive microfluidic circuit are configured to facilitate microfluidic flow properties. In particular, the microfluidic flow properties include that aqueous fluids to not mix with oleic fluids.

The expression "fluidicly connecting [...] with a or connectable to a reaction chamber" or "connected or connectable to a reaction chamber" or "connects or connectable to a reaction chamber" means according to the present invention that the second microfluidic channel may connect to a reaction chamber, which is already present in the substrate. It may connect directly to the reaction chamber or one or more (reservoir) chambers may be interposed. Alternatively the second microfluidic channel may connect to an outlet of the microfluidic circuit according to the present invention, wherein the outlet is connectable directly to a reaction chamber (RC) or is connectable to a reaction chamber (RC) interposed with one or more other chambers.

According to the first aspect of the present invention, the inventive microfluidic circuit for use in a microfluidic chip for isolating and purifying an analyte, preferably a nucleic acid, comprised in a biologic sample, is arranged in a substrate and comprises a first microfluidic channel (MC1) fluidicly connecting an outlet of a buffer chamber (BC) with an inlet of an elution chamber (EC). It further comprises a second microfluidic channel (MC2) fluidicly connecting an outlet of the elution chamber (EC) with an inlet of a waste chamber (WC) and with a or connectable to a reaction chamber (RC). The second microfluidic channel (MC2) is branched at a first junction (J1) into two downstream sub-channels (MC21, MC22) of a first generation. The first sub-channel (MC21) connects to the inlet of the waste chamber (WC) and the second sub-channel (MC22) connects or is connectable to the reaction chamber (RC). According to the present invention the second sub-channel (MC22) of first generation is branched at a second junction (J2) into two downstream sub-channels of second generation, wherein a first sub-channel (MC221) connects or is connectable to the reaction chamber (RC) and a second sub-channel (MC222) is connected to the buffer chamber (BC) or to a further buffer chamber (BC) and is configured to facilitate fluidic flow in at least part of the second sub-channel of first generation (MC22) and the second microfluidic channel (MC2) in direction to the outlet of the elution chamber (EC). According to the present invention one or more buffer chambers (BC) respectively connected or connectable to the elution chamber (EC) may be comprised in the inventive microfluidic circuit. Preferably the buffer chamber (BC) or more buffer chambers (BC) comprise(s) in use an elution buffer. According to the present invention, the buffer chamber(s) (BC) is/are preferably configured to pass the fluid in one flow direction. Such a configuration may include suitable syringes, which may be actuatable by pressure or electronic means.

Thus, in view of the second junction of the second microfluidic channel, the inventive microfluidic circuit is adapted to facilitate bidirectional fluidic flow at least between the outlet of the elution chamber (EC) to the second junction (J2), i.e. downstream of the elution chamber (EC). In view of this bidirectional fluidic flow properties, the inventive microfluidic circuit facilitates to remove any residual contaminant, which may impair the enzymatic reaction of the nucleic acids in the reaction chamber. Furthermore, the inventive microfluidic circuit facilitates the isolation and purification reaction by use of one waste chamber. The waste chamber may be in form of a void in a substrate, it may also effect removal of constituents from the microfluidic system by forming a suitable outlet to the environment or forming a suitable outlet connectable to a suitable reservoir means.

In view of the present invention, the respective microfluidic circuits, its channels, the elution chamber, the waste chamber, the reaction chamber and the capturing chamber are generally configured as suitable voids or recesses in a suitable substrate. The material of a suitable substrate may be liquid and/or gas, preferably liquid and gas impermeable. As a counter example, polydimethylsiloxane (PDMS) is not regarded a fluid and gas impermeable substrate, as the substrate will be gas permeable. Furthermore, the substrate material may preferably not interfere with the processing of the fluid. In addition or alternatively, the material of the substrate may facilitate in particular thermal and optical processing of the liquid, such as irradiation with electromagnetic waves, such as in the infrared and/or ultraviolet range and/or visible range and or optical inspection. Accordingly, the material of the basic substrate is preferably transparent to the respective irradiated and/or emitted wavelength, in order to reduce a negative impact on processing of the aliquot of fluid. In addition or alternatively, the material of the substrate may not interfere with magnetic field and ultrasound impulses. Thus, according to an additional or alternative embodiment of the present invention, the substrate material may be selected from the group consisting of suitable glass and suitable polymers, wherein the polymers are preferably selected from the group consisting of polycarbonate, cyclic olefin copolymer, polystyrene, cyclic olefin polymer or poly(methyl methacrylate). More preferably, the substrate material is selected from polycarbonate.

According to an alternative or additive embodiment of the first inventive aspect, the first microfluidic channel (MC1) is branched at a third junction (J3) into two sub-channels of first generation. A first sub-channel (MC11) connects to the inlet of the elution chamber (EC) and a second sub-channel (MC12) connects to the second sub-channel (MC222) of second generation of the second microfluidic channel (MC2) thereby bypassing the elution chamber (EC). According to this embodiment, the same buffer chamber can be used for the bidirectional flow.

According to an alternative or additive embodiment of the first inventive aspect, the first and second microfluidic channels (MC1, MC2) and their respective sub-channels as well as the elution chamber (EC) are configured to facilitate microfluidic flow properties including prevention of mixture of oil and aqueous fluids, preferably are configured to respectively have an inner diameter perpendicular to the flow direction of 1 mm² or less. As an example the inner diameter of a microfluidic channel may be 0.8 mm to 0.5 mm or less and the inner diameter of the elution chamber may be 0.5 mm to 2.0 mm or less. Such a configuration facilitates the use of a liquid oil phase, in particular of the elution buffer in order to further increase the removal of aqueous contamination agents, such as lysate agents and/or washing agents.

According to an alternative or additive embodiment of the first inventive aspect, the one or more buffer chambers (BC) is/are configured as a syringe adapted to operate in one fluidic flow direction. The fluidic flow direction is generally towards the inlet of the elution chamber (EC). In the case the first microfluidic channel bypasses the elution chamber (EC) and connects to the second sub-channel of the second generation of the second microfluidic channel, then the fluidic flow direction may also follow this channel connection towards the outlet of the elution chamber (EC).

According to an alternative or additive embodiment of the first inventive aspect, the fluidic flow direction is controlled by an arrangement of three or more fluidic flow control means, preferably two-state valves (also called on/off valves). These fluidic control means are generally independently actuatable to facilitate (unimpeded) fluidic flow in a microfluidic channel (on-status) or actuatable to stop / prevent fluidic flow in a microfluidic channel (off-status).The fluidic control means may be actuatable by electric means, pressure means or any other suitable means. Particularly preferred valves are disclosed in the European patent application 19 186 118.6. The respective disclosure concerning the microfluidic valve disclosed in European patent application 19 186 118.6 is incorporated herein by reference.

According to the present invention, the fluidic flow control means, preferably two-state valves (also called on/off valves) are used for routing fluid to different locations of the inventive microfluidic circuit.

According to an alternative or additive embodiment of the first inventive aspect, a fluidic flow control means (V1) is arranged to control fluidic flow in the first sub-channel (MC221) of first generation of the second microfluidic channel (MC2) connecting to the inlet of the waste chamber (WC), a further fluidic flow control means (V2) is arranged to control fluidic flow in the first sub-channel (MC221) of second generation of the second microfluidic channel (MC2) connecting or connectable to the reaction chamber (RC), and a further fluidic control means (V3) is arranged to control fluidic flow in the second sub-channel (MC222) of second generation of the second microfluidic channel (MC2). According to an alternative or additive embodiment, the two-state valve is preferably used a fluidic flow control means.

According to an alternative or additive embodiment of the first inventive aspect, the waste chamber (WC) represents either a void in the substrate or an outlet to the environment, wherein the outlet may be connectable to a waste reservoir of any shape.

All features and embodiments disclosed with respect to the first aspect of the present invention are combinable alone or in (sub-)combination with each of the further aspects of the present invention respectively including each of the preferred embodiments thereof, provided the resulting combination of features is reasonable to a person skilled in the art.

According to the second aspect of the present invention, the inventive microfluidic chip for isolating and purifying an analyte, preferably a nucleic acid, comprised in a biologic sample has a substrate comprising sample chamber (SC) fluidicly connected with an inlet of a capturing chamber (CC), characterized in that an outlet of the capturing chamber (CC) is fluidicly connected to the microfluidic circuit according to the first inventive aspect, wherein the outlet of the capturing chamber (CC) is connected with a third microfluidic channel (MC3) to a fourth junction (J4) of the first microfluidic channel (MC1). The fourth junction may be arranged between the buffer chamber (BC) and the third junction (J3) of the first microfluidic channel (MC1) or between the third junction (J3) of the first microfluidic channel (MC1) and the inlet of the elution chamber (EC).

According to an alternative or additive embodiment of the second inventive aspect, a fluidic flow control means (V4), preferably a two-state valve, is arranged to control fluidic flow in the third microfluidic channel (MC3) between the outlet of the capturing chamber (CC) and the fourth junction (J4) of the first microfluidic channel (MC1). The fluidic flow direction is towards the inlet of the elution chamber (EC).

According to an alternative or additive embodiment of the second inventive aspect, the capturing chamber (CC) comprises suitable magnetic capturing carriers adapted to bind isolated analyte, preferably nucleic acid, lysed from a biologic sample. According to an alternative or additive embodiment of the second inventive aspect, the suitable magnetic capturing carriers are selected from suitable bead, preferably microbeads, optionally wherein the beads / microbeads have the same or differing dimensions. Suitable capturing carriers include magnetic silica beads or magnetic silica powder. In case of nucleic acids as respective analytes, the magnetic capturing carriers, in particular magnetic silica beads / powders are negatively charged and, therefore, easily bind the negatively charged nucleic acids (suitable preparations are available by Merck under the MagPrep^{®} product line). The dimensions of the capturing carriers are generally adapted to facilitate transportation through the inventive microfluidic circuit. Preferably the diameter of suitable magnetic capturing carriers, in particular magnetic silica beads / microspheres / powder is in the range until 500 nm, preferably 50 to 400 nm, more preferably 100 to 200 nm.

According to an alternative or additive embodiment of the second inventive aspect, the capturing chamber (CC) is configured to have an inner volume to receive up to 5 ml, preferably between 1 to 4 ml, more preferably between 1.5 to 3 ml, in particular up to 2 ml of a fluid. In other words, the inner volume of the capturing chamber (CC) may exhibit in addition to the suitable amount of capturing carriers the respective volumes of fluid. Such a volume dimension facilitates a suitable binding of the analyte, preferably nucleic acid, with the capturing agents.

According to an alternative or additive embodiment of the second inventive aspect, the sample chamber (SC) is configured to receive an inner volume of up to 3 ml, preferably 0.5 ml to 2 ml, preferably up to 1 ml of a fluid. The sample chamber may also be configured to direct fluidic flow in one direction, preferably it may be configured as a syringe with fluidic flow in one direction.

According to an alternative or additive embodiment of the second inventive aspect, the microfluidic chip further comprises a lysis agent chamber (LC) and/or a dissolution agent chamber (DC) respectively in fluidic connection with the sample chamber (SC) and/or the capturing chamber (CC). In particular, the dissolution agent chamber (DC) may be fluidicly connected with the sample chamber (SC) and the lysis agent chamber (LC) may be fluidicly connected either with the sample chamber (SC) or with the capturing chamber (CC). Furthermore, the microfluidic chip may further comprise a washing agent chamber (WA) which may be in fluidic connection with the inlet of the elution chamber.

All features and embodiments disclosed with respect to the second aspect of the present invention are combinable alone or in (sub-)combination with each of the other aspects of the present invention respectively including each of the preferred embodiments thereof, provided the resulting combination of features is reasonable to a person skilled in the art.

According to the third aspect of the present invention, the inventive kit comprises or consists of the microfluidic chip according to second aspect of the present invention, and one or more, the same or different, suitable reaction agents selected from the group consisting of a reagent suitable for performing lysis of biologic sample (lysis agent); a reagent suitable for performing washing of lysed biologic cell fragments from the isolated analyte, preferably nucleic acid, respectively bound to at least part of the capturing carriers (washing agent); a reagent suitable for eluting the analyte, preferably isolated nucleic acid, from the capturing carriers (elution agent); a reagent suitable for suspending the sample (dissolution agent).

Generally the elution agent (synonym elution buffer) is suitable to elute the respective analyte, preferably nucleic acid, from the capturing carrier, e.g. buffer agents containing in particular Tris (tis(hydroxymethyl)aminomethane). In particular the buffer may be suitably adapted in its concentration and/or in its pH value with HCI to elute deoxyribo nucleic acids (DNA) or ribonucleic acids (RNA), wherein the RNA elution buffer may suitably exhibit a higher concentration of Tris and a higher pH value in comparison to DNA elution buffers. Suitable compositions are commercially available as Tris Base from Roth.

Generally, the lysis agent is suitable to lyse the biologic sample, such as cells, in particular human cells, bacterial cells or viral cells. Lysis agents suitable for bacterial cells may generally contain one or more constituents selected from the group consisting of salts, e.g. organic salts, such as Tris HCI, e.g. inorganic salts, such as NaCI; and detergents, e.g. anionic detergent SDS (sodium dodecyl sulfate/sulphate), non-ionic Triton X 100 with the following formula I, wherein n is 9 or 10 (MW: 647)

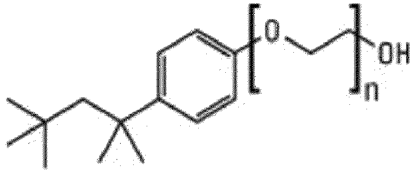

Alternatively, enzymatic lysis agents for bacterial lysis may contain one or more enzymes, such as selected from Mutanolysin, Trehalose (Proteinase A&A, Mutanolysin A&A, Trehalose Roth).

Furthermore, the lysis of bacterial cells may be conducted using a chemical mixture comprising a suitable alcohol including isopropanol, suitable salts and detergents.

Lysis agents suitable to destruct viral cells generally comprise Guanidinium thiocyanate and optionally buffer salts, such as Tris. The lysis agent may be adapted in view of the concentration of Guanidinium thiocyanate as well as of the pH value.

Suitable washing agents may generally comprise suitable buffer salts, such as Tris buffers. As with respect to the lysis agent, the washing agent may be adapted with respect to the respective analytes, in particular DNA or RNA with respect to the salt concentration and pH value. The washing agents may optionally contain further constituents such as reducing agents, e.g. dithiothreitol (DTT), in order to reduce disulfide bonds and to protect from oxidization.

All features and embodiments disclosed with respect to the third aspect of the present invention are combinable alone or in (sub-)combination with each of the other aspects of the present invention respectively including each of the preferred embodiments thereof, provided the resulting combination of features is reasonable.

According to the fourth aspect of the present invention, the inventive system configured to perform isolation and purification of an analyte, preferably nucleic acid, from a biologic sample, characterized in that it comprises or consists of a microfluidic chip according to second aspect of the present invention and a thermocycler.

The thermocycler (also known as a thermal cycler, PCR machine or DNA amplifier) is a device which comprises heating / cooling means, such as a thermal block or other heating / cooling means, to facilitate a polymerase chain reaction with nucleic acids, in particular the amplification of segments of nucleic acid, or other temperature-sensitive reactions, including restriction enzyme digestion or rapid diagnostics.

According to an alternative or additive embodiment of the second inventive aspect, the system further comprises or consists of a magnetic field excitation means arranged in use in such a distance to the elution chamber (EC) of the microfluidic chip that upon magnetic field excitation at least part of the magnetic capturing carriers form an aggregated magnetic conglomerate comprising analyte, preferably nucleic acid, bound thereto, further preferably located due to the magnetic field at a wall of the elution chamber (EC). Generally, the means of magnetic field excitation may comprise a permanent magnet, such as a neodymium magnet, that preferably can be moved relative to the microfluidic channel. Alternatively, an electromagnet may be selected as magnetic excitation means. The distance of the magnetic excitation means to the inventive microfluidic circuit is suitably chosen to exhibit the magnetic effect within the microfluidic channel and in particular the elution chamber. Accordingly the magnetic field excitation means is arranged suitably close to the inventive microfluidic circuit, preferably in a distance of less than 5 mm, more preferably less than 1 mm.

According to an alternative or additive embodiment of the second inventive aspect, the system further comprises an ultrasound excitation means arranged in use in such a distance to the elution chamber (EC) of the microfluidic chip that upon ultrasound impulse excitation the aggregated magnetic conglomerate is disaggregated at least in part into separate magnetic capturing carriers respectively with analyte, preferably nucleic acid, bound thereto so that the analyte, preferably nucleic acid, can be eluted from the magnetic capturing carriers. The means of ultrasound excitation may be chosen from a suitable ultrasonic transducer. The ultrasound excitation means is generally arranged in relation to the inventive microfluidic circuit in such a way that it may transmit in use respective acoustic waves through a suitable medium, such as a non-permeable substrate/membrane to the fluid located in use within the inventive microfluidic circuit, in particular the elution chamber thereof.

All features and embodiments disclosed with respect to the fourth aspect of the present invention are combinable alone or in (sub-)combination with each of the other aspects of the present invention respectively including each of the preferred embodiments thereof, provided the resulting combination of features is reasonable.

According to the fifth aspect of the present invention, the inventive process for isolating and purifying an analyte, preferably a nucleic acid, from a biological cell sample comprises or consists of the following steps:
Step a: Providing the system of fourth inventive aspect, providing a biological sample, providing a lysis agent, providing an elution agent and optionally providing a washing agent.
Step b: Loading the biological sample into the sample chamber (SC) of the microfluidic chip. The sample may be diluted with a suitable dilution agent.
Step c: Subsequently mixing the lysis agent with the optionally diluted biological sample thereby providing a fluidic mixture comprising lysed biologic sample fragments and isolated analyte, preferably nucleic acid.
Step d: Subsequently passing the fluidic mixture of step c) to the capturing chamber (CC) comprising the magnetic capturing carriers so that the isolated analyte, preferably nucleic acid, binds to at least part of the magnetic capturing carriers.
Step e: Subsequently passing the fluidic mixture of step d) via the third microfluidic channel (MC3) to the elution chamber (EC), wherein the magnetic field excitation means excites a suitable magnetic field so that at least part of the magnetic capturing carriers agglomerate as a magnetic conglomerate, which may be located due to the applied magnetic field at a wall of the elution chamber (EC).
Step f: Subsequently passing the elution agent from the buffer chamber (BC) via the elution chamber (EC) to the waste chamber (WC), wherein the magnetic field is maintained to keep the magnetic conglomerate located at the wall of the eluting chamber (EC).
Step g: Subsequently passing the elution agent from the buffer chamber (BC) to the second sub-channel (MC222) to facilitate fluidic flow in at least part of the second sub-channel of first generation (MC22) and the second microfluidic channel (MC2) towards the outlet of the elution chamber (EC) in order to remove remaining lysis agent from the second sub-channel of first generation (MC22), wherein the magnetic field is maintained to keep the magnetic conglomerate located at the wall of the eluting chamber (EC).
Step h: Subsequently passing the elution agent from the buffer chamber (BC) via the elution chamber (EC) to the waste chamber (WC) in order to remove the remaining lysis agent from the second micro fluidic channel (MC2), wherein the magnetic field is maintained to keep the magnetic conglomerate located at the wall of the eluting chamber (EC).
Step i: Subsequently removing the magnetic field and exciting the aggregated conglomerate of magnetic capture carriers with a suitable ultrasound impulse in order to separate the magnetic capturing carriers and thereby eluting the isolated analyte, preferably nucleic acid, from the capturing carriers.
Step j: Subsequently passing the elution agent from the buffer chamber via the elution chamber (EC) to the reaction chamber (RC) thereby moving the eluted analyte, preferably nucleic acid, to the reaction chamber (RC).

All features and embodiments disclosed with respect to the fifth aspect of the present invention are combinable alone or in (sub-)combination with each of the other aspects of the present invention respectively including each of the preferred embodiments thereof, provided the resulting combination of features is reasonable.

According to the sixth aspect of the present invention, the inventive use of a microfluidic circuit according to the first inventive aspect or a microfluidic chip according to the second inventive aspect or a kit according to the third inventive aspect or a system according to the fourth inventive aspect for isolating and purifying an analyte, preferably a nucleic acid, from a biological sample, preferably for conducting a biochemical assay selected from the group consisting of an enzymatic analysis of nucleic acids including DNA or RNA analysis, such as a polymerase chain reaction (PCR) and in particular a PCR with high-throughput sequencing.

All features and embodiments disclosed with respect to the sixth aspect of the present invention are combinable alone or in (sub-)combination with each of the other aspects of the present invention respectively including each of the preferred embodiments thereof, provided the resulting combination of features is reasonable to a person skilled in the art.

### DETAILED DESCRIPTION OF THE FIGURES:

Further characteristics and advantages of the present invention will ensue from the following description of embodiments of the inventive aspects with reference to the accompanying drawings.

As already discussed in the background part of the present invention, figures 1A and 1B represent schematic top views of part of a prior art microfluidic channel system at a junction connecting to the waste chamber (WC) and connecting to the reaction chamber (RC). These arrows in Figures 1A and 1B illustrate the fluidic flow direction of a lysis buffer (LB) at the junction connecting to the waste chamber (WC) and connecting to the reaction chamber (RC). As can be seen from Fig. 1A, part of the lysis buffer is forced into a part of the sub-channel (see distance d) connecting to the reaction chamber. Upon fluidic flow of the elution buffer (EB) the part of the lysis buffer located in the part of the sub-channel connecting to the reaction chamber (RC) is also transferred to the reaction chamber (RC) and may interfere with the enzymatic reaction of the analytes, preferably nucleic acids, as illustrated in Fig. 1B.

In order to reduce the lysis buffer or any other contamination during the reaction procedure, the present invention provides the following solution.

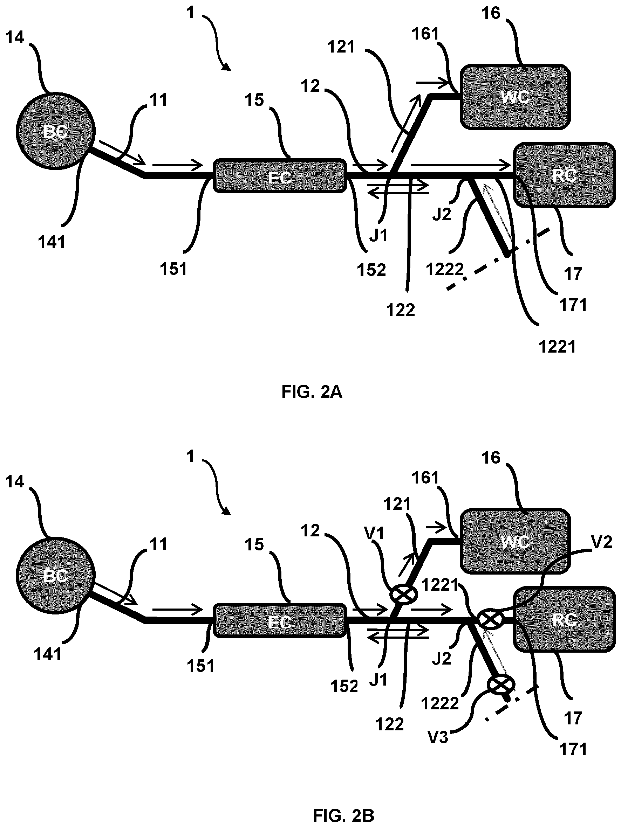

Figures 2A and 2B represent schematic top views of an inventive microfluidic circuit 1 comprising a buffer chamber 14 (BC), an elution chamber 15 (EC), a waste chamber 16 (WC) and a reaction chamber 17 (RC). In particular, the buffer chamber 14 (BC) may comprise in use the elution buffer (EB, not illustrated in Figures 2A, 2B). The outlet 141 of the buffer chamber 14 is connected via a first microfluidic channel with the inlet 151 of the elution chamber 15. The outlet 152 of the elution chamber 15 is connected to a second microfluidic channel which connects to the waste chamber 16 or alternatively to the reaction chamber 17. At junction J1, the second microfluidic channel is branched into two sub-channels of first generation, wherein the first sub-channel 121 connects to the waste chamber 16 and the second sub-channel 122 connects to the reaction chamber 17. The second sub-channel of first generation 122 is further branched at a second junction J2 into a further sub-channel of second generation 1221 connecting to the reaction chamber and a further sub-channel of second generation 1222. The sub-channel of second generation 1222 is connected to either the buffer chamber 14 (not illustrated in Figures 2A, 2B) or to a further buffer chamber (not illustrated in Figures 2A, 2B). In view of the fluidic flow of an elution buffer starting at the buffer chamber 14 or another buffer chamber, the elution buffer flows through the sub-channel of second generation 1222 in direction to the outlet 152 of elution chamber 15. The flow direction is illustrated by the respective arrows. This inventive arrangement of the microfluidic circuit facilitates the bidirectional flow properties downstream of the elution buffer in order to remove remaining fluidic contaminations, such as with lysis buffer and/or washing agents etc.. In Fig. 2B the fluidic flow control means, such as two-state valves V1, V2, V3 are illustrated, which control and direct the fluidic flow.

According to the present invention the microfluidic channels, including the first and second microfluidic channels (MC1, MC2) and their respective sub-channels as well as the elution chamber are configured to facilitate microfluidic flow properties including prevention of mixture of oil and aqueous fluids. According to an additional or alternative embodiment, the microfluidic channels, including the first and second microfluidic channels (MC1, MC2) and their respective sub-channels as well as the elution chamber are configured to respectively have an inner diameter perpendicular to the flow direction of 1 mm² or less. As an example the microfluidic channels have an inner diameter of 0.8 mm to 0.5 mm, whereas the elution chamber has an inner diameter of 0.5 mm to 2.0 mm.

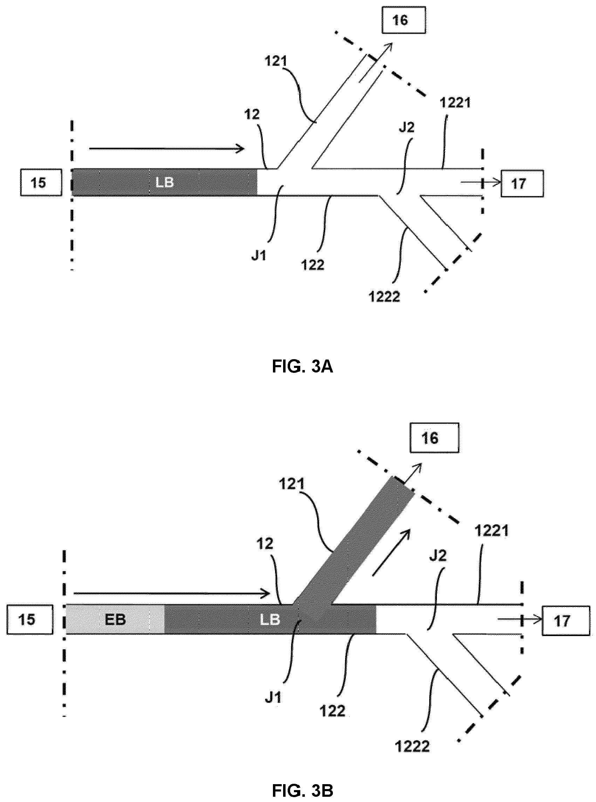

The fluidic flow properties are in particular illustrated in Figures 3A to 3F, which represent schematic top views of an exploded part of an inventive microfluidic circuit 1 in the area of the first and second junction (J1, J2) of the second microfluidic channel 12 respectively illustrating the fluidic flow condition of the elution buffer (EB) and the lysate agent buffer (LB). Figure 3A illustrates the situation wherein the lysate buffer (LB) is transferred through the elution chamber 15 into the second microfluidic channel 12.

According to Figure 3B the lysate buffer (LB) is already further transferred through the second microfluidic channel 12 and sub-channel 121 in direction to the waste chamber 16, wherein part of the lysis buffer (LB) is forced at the first junction J1 into part of the sub-channel 122. The elution buffer (EB) is already comprised in the second microfluidic channel 12.

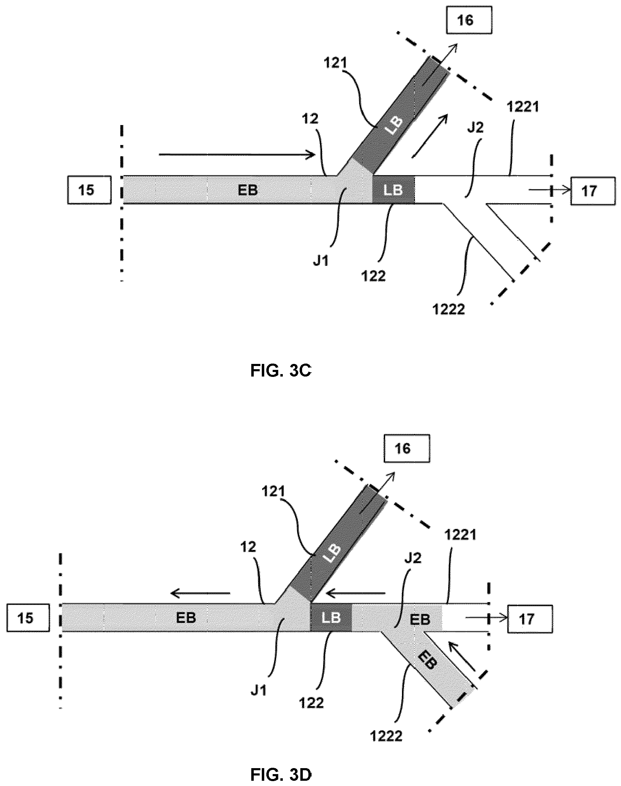

According to Figure 3C the elution buffer (EB) further propagates into direction of the waste chamber 16 so that the first junction J1 is completely filled with the elution buffer (EB).

Then, as illustrated in Figure 3D, the second sub-channel of second generation 1222 is filled with the elution buffer (EB), wherein the fluidic flow direction, which is depicted by the arrows is via the second junction J2 in direction to the outlet of the elution chamber 15.

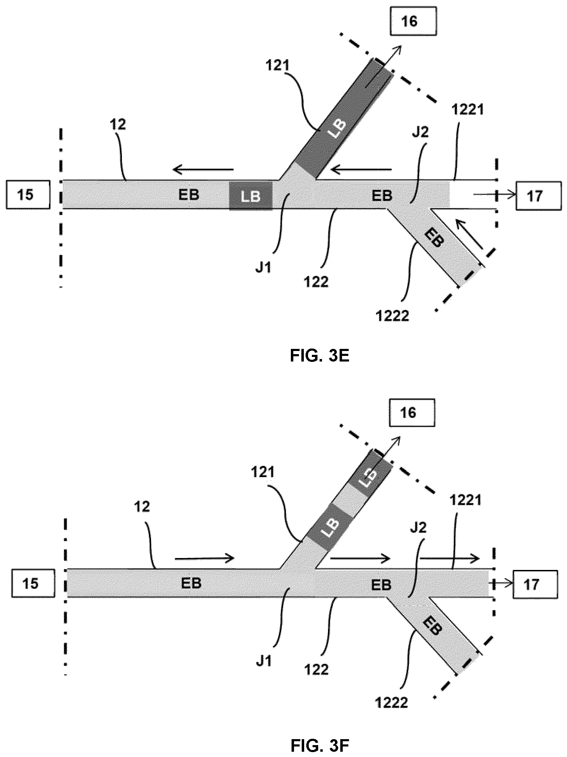

According to Figure 3E, this flow direction is continued so that the remaining lysate buffer (EB), which was located in the sub-channel 122 is moved via the first junction J1 in flow direction to the elution chamber 15 so that the junction J1 is preferably completely filled with elution buffer (EB).

Then the elution buffer (EB) is transferred from the buffer chamber 14 via the inlet of the elution chamber 15 (not illustrated) to the second microfluidic channel 12 and further through the first junction J1 to the waste chamber 17 so that the remaining lysate buffer is transferred through the sub-channel 121 to the waste chamber 16. Subsequently, the eluate buffer is transferred through the first junction J1 and the sub-channel 122 through the junction J2 and the sub-channel 1221 to the reaction chamber 17. Accordingly, the eluted analyte, preferably nucleic acid, is preferably free of the remaining contaminants.

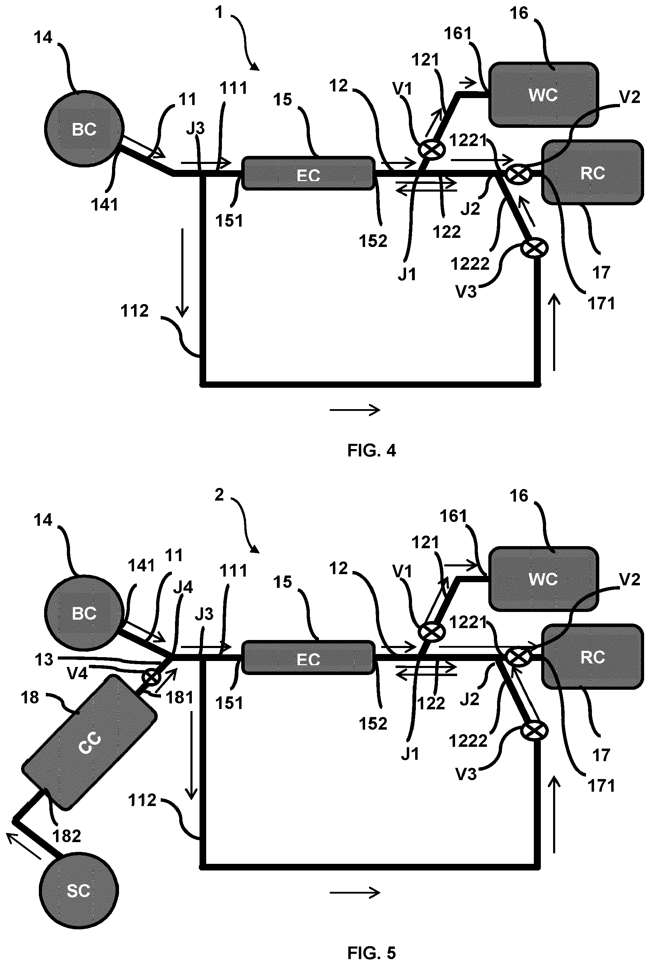

According to Figure 4, a schematic top view of an inventive microfluidic circuit 1 comprising a buffer chamber 14 (BC), an elution chamber 15 (EC), a waste chamber 16 (WC) and a reaction chamber 17 (RC) and a channel bypassing the elution chamber 15 (EC) is illustrated. The potential fluidic flow directions are illustrated with the arrows. Accordingly, the eluate buffer may be transferred from the buffer chamber 14 via the first microfluidic channel 11 through the third junction J3 and the sub-channel of first generation 111 to the inlet 151 of the elution chamber 15 and through the outlet 152 and the second microfluidic channel through the first junction J1 either via sub-channel 121 through fluid control means, preferably two-state valve V1 to the inlet 161 of the waste chamber 16. Alternatively, the elution buffer may propagate downstream of the elution chamber 15 via the first junction, the sub-channel of first generation 122 to the second junction J2 and the sub-channel of second generation 1221 through fluid control means, preferably two-state valve V2 to the inlet 171 of the reaction chamber 17. In order to facilitate the bidirectional flow, the elution buffer may propagate through the outlet of the buffer chamber 14 via the first microfluidic channel 11 to the third junction 3 to the sub-channel of first generation 112 via fluid control means, preferably two-state valve V3 to the sub-channel of second generation 1222 via the second junction J2, the sub-channel of first generation 122 via the first junction J1 and the second microfluidic channel 12 to the outlet 152 of the elution chamber 15.

According to Figure 5 a schematic top view of an inventive microfluidic chip 3 comprising a sample chamber 19 (SC), a capturing chamber 18 (CC) connected to the inventive microfluidic circuit 1 of Fig. 3 is illustrated. In addition to the inventive microfluidic circuit as illustrated in Figure 3, the microfluidic chip comprises the sample chamber 19 which is connected via a microfluidic channel to the inlet 182 of the capturing chamber 18. The capturing chamber preferably exhibits a greater volume than the elution chamber. Thereby the capturing procedure of the lysed analyte, preferably nucleic acid, can be facilitated with a higher volume of fluid in order to ensure that the major amount of isolated analyte, preferably nucleic acid, is bound to the capturing carriers preferably located in the capturing chamber 18. After binding of the analyte, preferably nucleic acid, the loaded capturing carriers bound with analyte, preferably nucleic acid, are then transferred through the third microfluidic channel 13 via a fourth fluid control means, preferably a two-state valve V4, through junction J4 via channels 11 and 111 to the inlet 151 of the elution chamber 15. The inner volume of the elution chamber 15 can be smaller than the inner volume of the capturing chamber 18, as the binding reaction between the analyte, preferably nucleic acid, and the capturing carriers already has taken place. The elution chamber 15 is in other words only relevant for removing the contaminants, such as lysate buffer and/or washing buffer from the fluidic system. In view of the smaller volume of the elution chamber 15 also a lower volume of washing elution buffer is necessary to remove all contaminants. In view of the inventive magnetic conglomeration of the analyte, preferably nucleic acid, loaded capturing carriers the amount of analyte, preferably nucleic acid, lost in the waste chamber could be reduced. Thus, the analytical precision could be increased in accordance with the present invention.

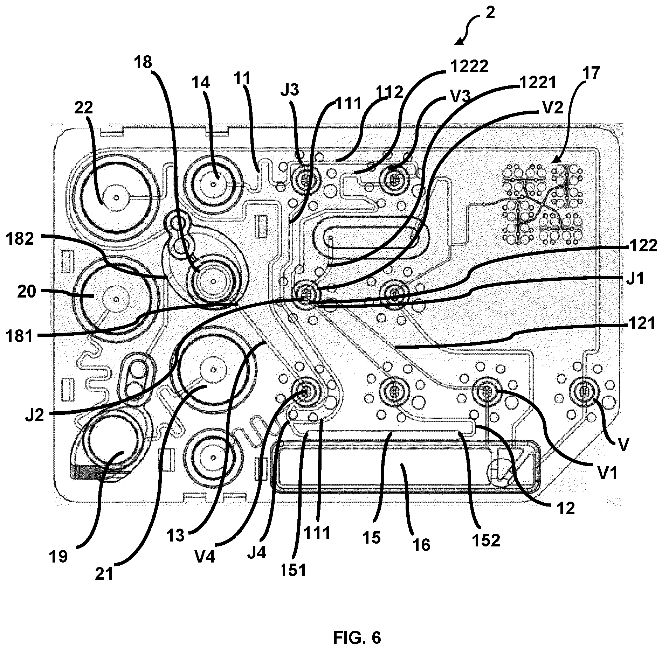

According to Figure 6 a design of an inventive microfluidic chip is illustrated, having a sample chamber 19 a lysis agent chamber 20 connected via a microfluidic channel to the sample chamber 19. Further it comprises a dissolution agent chamber 21 which is also connected via a microfluidic channel to the sample chamber 19. The sample chamber 19 is furthermore connected via a microfluidic channel to an inlet 182 of a capturing chamber 18. In use the capturing chamber 18 comprises suitable capturing means, such as magnetic capturing means, preferably magnetic (micro) beads of the same or different dimensions. In this capturing chamber 18 the isolated analyte, preferably nucleic acid, from the lysed biologic sample is bound to the capturing carriers. The analyte, preferably nucleic acid, loaded capturing carriers are then transferred through the outlet 181 via the third microfluidic channel 13 and a fourth fluid control means, such as a suitable two-state valve, via the fourth junction J4 to the inlet 151 of the elution chamber 15. The outlet 152 of the elution chamber 15 is connected via the second microfluidic channel 12 to a first junction J1 branching the second microfluidic channel into sub-channel of first generation 121, which connects via a first fluid control means, preferably a two-state valve to the waste chamber 16. Furthermore, the buffer chamber 14 connects via the first microfluidic channel 11 to the third junction J3 to sub-channel 111 to the inlet 151 of the elution chamber 15. Furthermore, the first microfluidic channel 11 connects alternatively through junction J3 to sub-channel of first generation 112, which connects via fluidic control means, preferably two-state valve V3 to the second sub-channel of second generation 1222, which connects via junction J2 to sub-channel of first generation 122. Alternatively, sub-channel of first generation 122 connects via junction J2 and fluid control means, preferably two-state valve V2 to sub-channel 1221 of second generation, which connects interposed by a reservoir chamber to a plurality of reaction chamber 17. The microfluidic chip contains further fluid control means, preferably two-state valves, illustrated also as V, as well as other chambers, such as a washing agent chamber 22, which is connected via a microfluidic channel to the third microfluidic channel 13.

All of the features disclosed with respect to the accompanying figures can alone or in any sub-combination be combined with features of the three aspects of the present invention including features of preferred embodiments thereof, provided the resulting feature combination is reasonable to a person skilled in the art.

### Reference numerals:

1 microfluidic circuit
10 microfluidic channel (MC)
11 first microfluidic channel (MC1)
111 sub-channel of first generation (MC11) connects to the inlet 151 of the elution chamber 15 (EC)
112 sub-channel of first generation (MC12) connects to the second sub-channel 1222 (MC222) of second generation of the second microfluidic channel 12 (MC2)
12 second microfluidic channel (MC2)
121 sub-channel of first generation (MC21) connecting to inlet 161 of waste chamber 16 (WC)
122 sub-channel of first generation (MC22) connecting to inlet 171 of reaction chamber 17 (RC)
1221 sub-channel of second generation (MC221) connects or is connectable to the reaction chamber (RC)
1222 sub-channel of second generation (MC222)
13 third microfluidic channel (MC3)
14 buffer chamber (BC)
141 outlet of buffer chamber (BC)
15 elution chamber (EC)
151 inlet of an elution chamber (EC)
152 outlet of an elution chamber 15 (EC)
16 waste chamber (WC)
161 inlet of a waste chamber 16 (WC)
17 reaction chamber (RC)
171 inlet of reaction chamber 17 (RC)
18 capturing chamber (CC)
181 outlet of capturing chamber 18 (CC)
182 inlet of capturing chamber 18 (CC)
19 sample chamber (SC)
191 outlet of sample chamber 19 (SC)
20 lysis agent chamber (LC) in fluidic connection with the sample chamber 19 (SC) or the capturing chamber 18 (CC)
21 dissolution agent chamber (DC) in fluidic connection with the sample chamber 19 (SC) and/or the capturing chamber 17 (CC)
22 washing agent chamber (WA) in fluidic connection with the third microfluidic channel 13
J junction branching a microfluidic channel
J1 junction branching second microfluidic channel into two downstream sub-channels (121, 122) of a first generation
J2 junction branching sub-channel 122 of first generation into two downstream sub-channels 1221 and 1222 of second generation
J3 junction branching first microfluidic channel 11 into two downstream sub-channels (111, 112) of a first generation
J4 junction connecting third microfluidic channel 13 (MC3) with first microfluidic channel 11
V fluidic flow control means, preferably two-state valve, arranged to control fluidic flow in a microfluidic channel
V1 fluidic flow control means, preferably two-state valve, arranged to control fluidic flow in the sub-channel of first generation 121 (MC21) of the second microfluidic channel 12 (MC2)connecting to the inlet 161 of the waste chamber 16 (WC)
V2 fluidic flow control means, preferably two-state valve, arranged to control fluidic flow in the sub-channel of second generation 1221 (MC221) of the second microfluidic channel 12 (MC2) connecting or connectable to the inlet 171 of the reaction chamber 17 (RC)
V3 fluidic flow control means, preferably two-state valve, arranged to control fluidic flow in the sub-channel of second generation 1222 (MC222) of the second microfluidic channel 12 (MC2)
V4 fluidic flow control means, preferably two-state valve, arranged to control fluidic flow of the third microfluidic channel 13 (MC3)

## Claims

1. A microfluidic circuit for use in a microfluidic chip for isolating and purifying an analyte, preferably a nucleic acid, comprised in a biologic sample, wherein the microfluidic circuit is arranged in a substrate and comprises a first microfluidic channel (MC1) fluidicly connecting an outlet of a buffer chamber (BC) with an inlet of an elution chamber (EC) and a second microfluidic channel (MC2) fluidicly connecting an outlet of the elution chamber (EC) with an inlet of a waste chamber (WC) and with a or connectable to a reaction chamber (RC), wherein the second microfluidic channel (MC2) is branched at a first junction (J1) into two downstream sub-channels (MC21, MC22) of a first generation, wherein the first sub-channel (MC21) connects to the inlet of the waste chamber (WC) and the second sub-channel (MC22) connects or is connectable to the reaction chamber (RC), **characterized in that** the second sub-channel (MC22) of first generation is branched at a second junction (J2) into two downstream sub-channels of second generation, wherein a first sub-channel (MC221) connects or is connectable to the reaction chamber (RC) and a second sub-channel (MC222) is connected to the buffer chamber (BC) or to a further buffer chamber (BC) and is configured to facilitate fluidic flow in at least part of the second sub-channel of first generation (MC22) and the second microfluidic channel (MC2) in direction to the outlet of the elution chamber (EC).

2. The microfluidic circuit according to claim 1, wherein the first microfluidic channel (MC1) is branched at a third junction (J3) into two sub-channels of first generation, wherein a first sub-channel (MC11) connects to the inlet of the elution chamber (EC) and a second sub-channel (MC12) connects to the second sub-channel (MC222) of second generation of the second microfluidic channel (MC2) thereby bypassing the elution chamber (EC).

3. The microfluidic circuit according to claim 1 or 2, wherein the first and second microfluidic channels (MC1, MC2) and their respective sub-channels as well as the elution chamber are configured to facilitate microfluidic flow properties including prevention of mixture of oil and aqueous fluids, preferably are configured to respectively have an inner diameter perpendicular to the flow direction of 1 mm² or less.

4. The microfluidic circuit according to any one of claims 1 to 3, wherein the buffer chamber (BC) is configured as a syringe adapted to operate in one direction.

5. The microfluidic circuit according to any one of claims 1 to 4, wherein the fluidic flow direction is controlled by an arrangement of three or more fluidic flow control means, preferably two-state valves, independently actuatable to facilitate fluidic flow in a microfluidic channel (on-status) or actuatable to stop fluidic flow in a microfluidic channel (off-status).

6. The microfluidic circuit according to claim 5, wherein a fluidic flow control means (V1) is arranged to control fluidic flow in the first sub-channel (MC221) of first generation of the second microfluidic channel (MC2) connecting to the inlet of the waste chamber (WC), a further fluidic flow control means (V2) is arranged to control fluidic flow in the first sub-channel (MC221) of second generation of the second microfluidic channel (MC2) connecting or connectable to the reaction chamber (RC), and a further fluidic control means (V3) is arranged to control fluidic flow in the second sub-channel (MC222) of second generation of the second microfluidic channel (MC2).

7. The microfluidic circuit according to any one of the preceding claims, wherein the waste chamber (WC) represents either a void in the substrate or an outlet to the environment, wherein the outlet may be connectable to a waste reservoir of any shape.

8. A microfluidic chip for isolating and purifying an analyte, preferably a nucleic acid, comprised in a biologic sample having a substrate comprising sample chamber (SC) fluidicly connected with an inlet of a capturing chamber (CC), **characterized in that** an outlet of the capturing chamber (CC) is fluidicly connected to the microfluidic circuit according to any one of claims 1 to 6, wherein the outlet of the capturing chamber (CC) is connected with a third microfluidic channel (MC3) to a fourth junction (J4) of the first microfluidic channel (MC1).

9. The microfluidic chip according to claim 8, wherein a fluidic flow control means (V4), preferably a two-state valve, is arranged to control fluidic flow in the third microfluidic channel (MC3) between the outlet of the capturing chamber (CC) and the third junction (J3) of the first microfluidic channel (MC1).

10. The microfluidic chip according to claim 8 or 9, wherein the capturing chamber (CC) comprises suitable magnetic capturing carriers adapted to bind isolated analyte, preferably nucleic acid, lysed from a biologic sample, preferably comprises suitable magnetic microbeads optionally having the same or differing dimensions.

11. The microfluidic chip according to any one of claims 8 to 10, wherein the capturing chamber (CC) is configured to have an inner volume to receive up to 5 ml, preferably between 1 to 4 ml, more preferably between 1.5 to 3 ml, in particular up to 2 ml of a fluid.

12. The microfluidic chip according to any one of claims 8 to 11, wherein the sample chamber (SC) is configured to receive an inner volume of up to 3 ml, preferably 0.5 ml to 2 ml, preferably up to 1 ml of a fluid.

13. The microfluidic chip according to claim 11 or 12, wherein the microfluidic chip further comprises a lysis agent chamber (LC) and/or a dissolution agent chamber (DC) respectively in fluidic connection with the sample chamber (SC) or the capturing chamber (CC).

14. A kit comprising or consisting of the microfluidic chip according to any one of claims 7 to 13, and one or more, the same or different, suitable reaction agents selected from the group consisting of a reagent suitable for performing lysis of biologic sample (lysis agent); a reagent suitable for performing washing of lysed biologic cell fragments from the isolated analyte, preferably nucleic acid, respectively bound to at least part of the capturing carriers (washing agent); a reagent suitable for eluting the isolated analyte, preferably nucleic acid, from the capturing carriers (elution agent); a reagent suitable for suspending the sample (dissolution agent).

15. A system configured to perform isolation and purification of an analyte, preferably nucleic acid, from a biologic sample, **characterized in that** it comprises or consists of a microfluidic chip according to any one of claims 7 to 13 and a thermocycler.

16. The system according to claim 15, further comprising or consisting of a magnetic field excitation means arranged in use in such a distance to the elution chamber (EC) of the microfluidic chip that upon magnetic field excitation at least part of the magnetic capturing carriers form an aggregated magnetic conglomerate comprising analyte, preferably nucleic acid, bound thereto, preferably located due to the magnetic field at a wall of the elution chamber (EC).

17. The system according to claim 16, further comprising an ultrasound excitation means arranged in use in such a distance to the elution chamber (EC) of the microfluidic chip that upon ultrasound impulse excitation the aggregated magnetic conglomerate is disaggregated at least in part into separate magnetic capturing carriers respectively with analyte, preferably nucleic acid, bound thereto so that the analyte, preferably nucleic acid, can be eluted from the magnetic capturing carriers.

18. A process for isolating and purifying an analyte, preferably a nucleic acid, from a biological cell sample comprising or consisting of the following steps:
a. Providing the system of any one of claims 15 to 17, providing a biological sample, providing a lysis agent, providing an elution agent and optionally providing a washing agent,
b. Loading the biological sample into the sample chamber (SC) of the microfluidic chip,
c. Subsequently mixing the lysis agent with the biological sample thereby providing a fluidic mixture comprising lysed biologic sample fragments and isolated analyte, preferably nucleic acid,
d. Subsequently passing the fluidic mixture of step c) to the capturing chamber (CC) comprising the magnetic capturing carriers so that the isolated analyte, preferably nucleic acid, binds to at least part of the magnetic capturing carriers,
e. Subsequently passing the fluidic mixture of step d) via the third microfluidic channel (MC3) to the elution chamber (EC), wherein the magnetic field excitation means excites a suitable magnetic field so that at least part of the magnetic capturing carriers agglomerate as a magnetic conglomerate, which is located due to the applied magnetic field at a wall of the elution chamber (EC),
f. Subsequently passing the elution agent from the buffer chamber (BC) via the elution chamber (EC) to the waste chamber (WC), wherein the magnetic field is maintained to keep the magnetic conglomerate located at the wall of the eluting chamber (EC),
g. Subsequently passing the elution agent from the buffer chamber (BC) to the second sub-channel (MC222) to facilitate fluidic flow in at least part of the second sub-channel of first generation (MC22) and the second microfluidic channel (MC2) towards the outlet of the elution chamber (EC) in order to remove remaining lysis agent from the second sub-channel of first generation (MC22), wherein the magnetic field is maintained to keep the magnetic conglomerate located at the wall of the eluting chamber (EC),
h. Subsequently passing the elution agent from the buffer chamber (BC) via the elution chamber (EC) to the waste chamber (WC) in order to remove the remaining lysis agent from the second micro fluidic channel (MC2), wherein the magnetic field is maintained to keep the magnetic conglomerate located at the wall of the eluting chamber (EC),
i. Subsequently removing the magnetic field and exciting the aggregated conglomerate of magnetic capture carriers with a suitable ultrasound impulse in order to separate the magnetic capturing carriers and thereby eluting the isolated analyte, preferably nucleic acid, from the capturing carriers, and
j. Subsequently passing the elution agent from the buffer chamber via the elution chamber (EC) to the reaction chamber (RC) thereby moving the eluted analyte, preferably nucleic acid, to the reaction chamber (RC).

19. Use of a microfluidic circuit according to any one of claims 1 to 6 or a microfluidic chip according to any one of claims 7 to 13 or a kit according to claim 14 or a system according to claim 15 to 18 for isolating and purifying an analyte, preferably a nucleic acid, from a biological sample, preferably for conducting a biochemical assay selected from the group consisting of an enzymatic analysis of analytes, preferably nucleic acids, including DNA or RNA analysis, such as a polymerase chain reaction (PCR) and in particular a PCR with high-throughput sequencing.
